# EUROPEAN PATENT SPECIFICATION

(11) **EP 3 302 403 B1**
(45) Date of publication and mention of the grant of the patent: **07.11.2018**
(21) Application number: 16723766.8
(22) Date of filing: 20.05.2016
(51) Int. Cl.: A61K 8/11, A61K 8/25, A61K 8/891, A61K 8/58, A61K 8/35, A61K 8/37, A61Q 17/04

(54) **A PHOTO-PROTECTIVE PERSONAL CARE COMPOSITION**
KÖRPERPFLEGEZUSAMMENSETZUNG MIT LICHTSCHUTZ
COMPOSITION DE SOINS PERSONNELS PHOTOPROTECTEURS

(30) Priority: 27.05.2015 WO PCT/CN2015/079925; 09.07.2015 EP 15176061
(43) Date of publication of application: 11.04.2018
(73) Proprietor: Unilever NV, 3013 AL Rotterdam (NL); Unilever PLC, London, Greater London EC4Y 0DY (GB)
(72) Inventor: LOU, Anjing, Trumbull Connecticut 06611 (US); MOADDEL, Teanoosh, Trumbull Connecticut 06611 (US); SONG, Wenhui, Shanghai 200335 (CN); ZHAO, Wei, Shanghai 200335 (CN)
(74) Representative: Corsten, Michael Allan
(86) International application number: PCT/EP2016/061378
(87) International publication number: WO 2016/188885

(56) References cited:
- EP-A1- 1 803 750
- WO-A1-2004/105712
- WO-A1-2007/000316
- WO-A1-2008/022946
- WO-A1-2013/117449
- WO-A2-2009/042535

## Description

### Field of the invention

The invention relates to a photo-protective personal care composition, especially to one that provides enhanced stability of a dibenzoylmethane UVA sunscreen when a cinnamic acid UVB sunscreen is also present. The invention further relates to a photo-protective composition that exhibits high SPF and UVAPF values.

### Background of the invention

Solar radiation includes about 5 % ultraviolet (UV) radiation, wavelength of which is between 200 nm and 400 nm. It is further classified into three regions: from 320 to 400 nm (UV-A), 290 to 320 nm (UV-B) and from 200 to 290 nm (UV-C). Exposure to UV-A and UV-B radiation for short period is known to cause reddening of the skin and localized irritation. Continued and prolonged exposure can lead to sunburn, melanoma and formation of wrinkles. It is also reported that UV radiation causes significant damage to hair. Therefore people desire to protect their skin and hair from the harmful effects of both UV-A and UV-B radiation.

Various cosmetic preparations have been reported for preventing and/or protecting the skin from harmful effects of ultraviolet radiation. These cosmetic compositions usually comprise different types of organic sunscreen agents, especially ones capable of absorbing the UV-A and/or UV-B radiation present in the sun's rays. Thus, both UV-A and UV-B sunscreens are usually incorporated in photo-protective compositions so as to provide protection over the entire range of UV radiation.

A commonly used organic sunscreen of the UVA type is a compound of the dibenzoylmethane group of which 4-t-butyl-4'-methoxydibenzoylmethane sold as Parsol 1789™ (or sold as Avobenzone™), is widely used in personal care compositions. The problem associated with this sunscreen is its relatively unstable nature. When this compound is incorporated in a sunscreen composition and this composition is applied on a substrate (e.g. skin) and exposed to sunlight, the UVA sunscreen degrades rapidly in a matter of a half hour to about two hours depending on the intensity of the radiation. The stability is seen to be further reduced when UVB sunscreen especially of the cinnamic acid class are incorporated in the composition. Efforts have been made to add some external molecule, like Octocrylene™, to improve the stability of Parsol 1789 in sunscreen formulations e.g. as disclosed in EP0514491. Investigations are ongoing throughout the world to find other alternatives to improve the stability and the photo-protection efficiency of Parsol 1789™ in such compositions.

The present applicants have other patents e.g. EP2555744 and EP2632418 which disclose natural actives like theaflavins and gingerol for solving the problem of photostability of the dibenzoylmethane based UVA sunscreen in a personal care composition. There is still a need in the art to prepare sunscreen compositions where the UVA sunscreen active is better stabilized, while at the same time using actives which are conventionally used in personal care and therefore are widely available and thus affordable at low cost to the consumer.

The present inventors have by way of extensive experimentation developed a novel composition where the UVA sunscreen of the dibenzoylmethane class when entrapped in a matrix comprising a specific silica and a specific silicone which when combined with a gel made of a silicone elastomer and a volatile silicone wherein a UVB sunscreen of the cinnamate class is entrapped, the UVA sunscreen maintains its activity for long time on exposure to sunlight, thereby providing high sun-protection as measured using SPF (sun protection factor) and UVAPF (UVA protection factor) methods.

It is therefore an object of the present invention to obviate at least some drawbacks of the prior art and provide a photo-protective personal care composition having high UVA efficacy for long time after it is exposed to sunlight.

Another object of the present invention is to provide longer term UVA sunscreen efficacy using a unique combination of well-known personal care ingredients thereby ensuring low cost to the consumer.

It is yet another object of the present invention to provide for a composition that exhibits higher SPF and UVAPF as compared to a conventional composition comprising the same UVA and UVB sunscreen actives.

### Summary of the invention

The first aspect of the present invention relates to a photo-protective personal care composition comprising
(a) 0.1 to 10 % by weight of a UVA sunscreen of the dibenzoylmethane class entrapped in a matrix comprising hydrophobically modified silica and amino functionalized silicone; and
(b) 0.1 to 10 % by weight of a UVB sunscreen of the cinnamate class entrapped in a gel comprising silicone elastomer and a volatile silicone, wherein (a), (b), silicone elastomer and volatile silicone are as defined in the appended claims.

The second aspect of the present invention relates to a method of enhancing the stability of UVA sunscreens of the dibenzoyl methane class in a composition comprising a UVB sunscreen of the cinnamate class, as specified in appended claim 9, comprising the steps of
(i) entrapping said UVA sunscreen in a matrix comprising hydrophobically modified silica and amino functionalized silicone;
(ii) entrapping said UVB sunscreen in a gel comprising silicone elastomer and a volatile silicone; and
(iii) mixing said matrix with said gel.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

By "A photo-protective personal care Composition" as used herein, is meant to include a composition for topical application to sun-exposed areas of the skin and/or hair of mammals, especially humans. Such a composition may be generally classified as leave-on or rinse off but is preferably of the leave on type. The composition may also be formulated into a product which is applied to a human body for also improving appearance, cleansing, odor control or general aesthetics. The composition of the present invention can be in the form of a liquid, lotion, cream, foam, scrub, gel, soap bar or toner, or applied with an implement or via a face mask or a pad. Non-limiting examples of such compositions include leave-on skin lotions, creams, antiperspirants, deodorants, lipsticks, foundations, mascara, sunless tanners and sunscreen lotions and wash-off shampoos, conditioners, shower gels, or toilet bars. The composition of the present invention is preferably a leave-on composition. "Skin" as used herein is meant to include skin on the face and body (e.g., neck, chest, back, arms, underarms, hands, legs, buttocks and scalp) and especially to the sun exposed parts thereof. The composition of the invention is also of relevance to applications on any other keratinous substrates of the human body other than skin e.g. hair where products may be formulated with specific aim of providing photo-protection.

By 'A High SPF composition' is meant a composition that has an SPF higher than 20, preferably higher than 30. By 'A High UVAPF composition' is meant a composition that has a UVAPF equal to or higher than 6, and more preferably higher than 10.

The SPF and UVAPF are measured using the standard protocols ISO/WD 4445 and ISO/CD 24443 respectively.

The present invention relates to a photo-protective personal care composition comprising a UVA sunscreen of the dibenzoylmethane class and a UVB sunscreen of the cinnamate class. The UVA sunscreen of the dibenzoylmethane group is preferably 4-tert-butyl-4'-methoxydibenzoylmethane. The UVA sunscreen of the dibenzoylmethane class is entrapped in a matrix comprising hydrophobically modified silica and amino functionalized silicone. The UVA sunscreen is present in 0.1 to 10 %, preferably 0.1 to 5 %, more preferably 0.4 to 3 % by weight of the composition. Similar compositions ar disclosed in WO 2004/105712 A1 (MERCK PATENT GMBH [DE]; CHAUDHURI RATAN [US]) 9 December 2004 (2004-12-09).

Without wishing to be bound by any theory the inventors believe that the problem to be solved lies in drawing a very fine balance between encapsulating the UVA and UVB sunscreens in relatively rigid capsules (where the UV efficacy is compromised) and including them in highly labile matrices where sunscreen efficacy is high but the UVA and UVB sunscreens diffuse towards each other in the packaging or after topical application to interact with each other thereby making the UVA sunscreen unstable. The present invention, by way of entrapping the UVA sunscreen in a matrix of hydrophobic silica and amino functionalized silicone, and by way of entrapping the UVB sunscreen in a gel of silicone elastomer in a volatile silicone provides the ideal balance between entrapment while ensuring desired sun screening efficacy thereby achieving the benefits of the present invention.

By "amino functionalized silicone" is meant a silicone containing at least one primary, secondary or tertiary amine group, or a quaternary ammonium group.
The primary, secondary, tertiary and/or quaternary amine groups may either form part of the main polymer chain or more preferably be carried by a side or pendant group carried by the polymeric backbone. Suitable amino functionalized silicone polymers for use with the invention are described in US 4 185 087.
Amino functionalized silicones suitable for use in the invention will typically have a mole % amine functionality in the range of from about 0.1 to about 8.0 mole %, preferably from about 0.1 to about 5.0 mole %, most preferably from about 0.1 to about 2.0 mole %. In general the amine concentration should not exceed about 8.0 mole %.

In a preferred embodiment, the functionalized polymers are of the amodimethicone having the general formula: 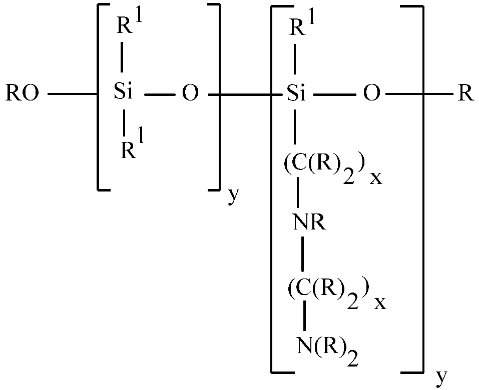
where each R is independently H, or a C₁₋₄ alkyl, preferably H;
each R¹ is independently OR or a C₁₋₄ alkyl; and
each x is independently an integer from 1 to 4 and each y is greater than zero and independently an integer to yield a polymer having a molecular weight from 500 to 1 million, and preferably from 750 to 25,000, and most preferably from 1,000 to 15,000 Daltons.

Particularly preferred are silicones comprising an amino glycol copolymer. Especially preferred is Bis (C₁₃-C₁₅ alkoxy) PG amodimethicone such as DC 8500 by Dow Corning.

It is also within the scope of this invention for the functionalized polymer to comprise trimethylsilylamodimethicone.

Preferably the level of amino functionalized silicone polymers make up from 1 to 15 wt%, preferably 3 to 10 % by total weight of the composition.

The compositions of the invention comprise hydrophobically modified silica (silicone dioxide). It is particularly preferred if the hydrophobically modified silica comprises the hydrophobic group of formula: 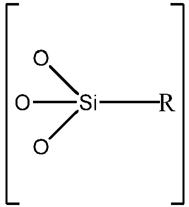 in which R is a C₄ to C₁₈ alkyl group.

Particularly preferred is the hydrophobically modified silicone dioxide comprising:

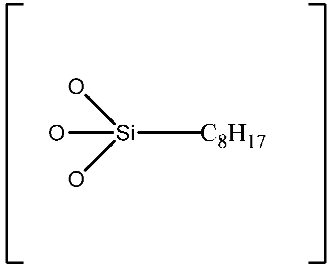

Preferred silicas are described in US7282236 and made commercially available from suppliers like Evonik Degussa GmbH under the names Aerosil R812, R202 and R805. Silica having a C₄ to C₁₅ alkyl group attached to the silane are preferred. Particularly preferred is octylsilane comprising the group represented by the formula above (sold under the name Aerosil R805).

The hydrophobically modified silica preferably has a primary particle size (in its dry state) from 1 nm to 100 nm, more preferably from 5 nm to 70 nm.

Composition of the invention preferably comprises from 0.5 to 8%, more preferably 1 to 6 % hydrophobically modified silica by total weight of the composition.

Preferably, the weight ratio of hydrophobically modified particle to amino functionalized silicone is from 3:1 to 1:50, more preferably from 1:1 to 1:10.

The UVB sunscreen of the cinnamate class, as per the present invention is preferably 2-ethylhexyl-p-methoxycinnamate.The UVB sunscreen is present in 0.1 to 10 %, preferably 1 to 8 %, more preferably 2 to 6 % by weight of the composition.

According to another aspect of the present invention the composition is prepared using a process comprising the steps where the gel comprising the silicone elastomer and the volatile silicone is prepared by a process of polymerization which comprises the steps of (a) mixing a hydride silicone copolymer with a vinyl terminated silicone polymer in the presence of the volatile silicone compound and a transition metal catalyst and (b) heating the mixture to a temperature of 40 to 90 °C to form the desired gel. As a preferred aspect, the UVB sunscreen of the cinnamate class is included in the step (a) or step (b) before the end of the polymerization process. Thus the UVB sunscreen is included in the gel during the insitu polymerization reaction, thus ensuring uniform entrapment of the UVB sunscreen in the gel.

A typical process for carrying out the process of polymerization of the gel comprising the silicone elastomer and the volatile silicone by including the UVB sunscreen therein, is as follows:
Firstly, the following ingredients are taken, in no particular preference of order and mixed thoroughly to form a homogeneous fluid blend at room temperature: silicone hydride copolymer, vinyl terminated silicone polymer, the volatile silicone, the UVB sunscreen and preferably its solubility enhancer such as Silsoft34™. The temperature of the mixture is then raised to about 35 °C and then the desired (platinum based) catalyst is added with constant stirring. The reaction mixture is then heated to about 65 °C with constant stirring until the reaction is completed and the primary elastomer is prepared, generally in about an hour, after which the mixture is cooled to room temperature (of about 25 °C). Finally the elastomer is diluted with desired amount of volatile silicone at room temperature to a gel composition with desired rheological properties.

The composition preferably comprises 3 to 40% silicone elastomer by weight of the composition. Silicone elastomers differ from linear polymers because of cross-linking. Many silicone elastomers are made from linear silicone polymers that contain reactive sites along the polymer chain. Elastomers have very different physical and chemical properties from linear polymers, and the properties of elastomers depend very much on the number of cross-links. An elastomer with a relatively small number of cross-links will be very soft and will swell significantly in the presence of a compatible solvent. As the number of cross-links increases, the hardness of the elastomer increases, and the elastomer will swell to a lesser extent in the presence of a solvent.

The volatile silicone for incorporation in the gel preferably has a vapor pressure value at 25°C of 2.6 to 1400 Pa, and a heat of vaporization value of 21 to 84 kJ/mole. The volatile silicone meeting the above specification of vapour pressure and heat of vaporization ensures that the heat absorbed from the substrate (e.g. skin) is such as to have the desired sensory perception of cooling while contributing to the desired UV protection and composition stability. The volatile silicone is preferably selected from octa methyl cyclo tetra siloxane, deca methyl cyclo penta siloxane, dodecamethyl cyclo hexa siloxane, blends of methyl trimethicone and dimethicone and mixtures thereof. The composition preferably comprises 3 to 60%, preferably 10 to 45 %, more preferably in 15 to 35 % volatile silicone by weight of said composition.

The preferred commercially available volatile silicones are (a) decamethyl cyclopentasiloxane (commercially known as D₅ available from Dow Corning DC245); (b) dodecamethyl cyclohexasiloxane (commercially known as D₆ and available from Dow corning); (c) TMF-1.5 (INCI: Methyl Trimethicone); (d) DM-Fluids-A₆ (INCI: Dimethicone); (e) DM-Fluid-2cs (INCI: Dimethicone); (f) SF1000N1 (INCI: Trisiloxane (1 cSt); (g) SF1000N1.5 (INCI: Dimethicone (1.5cSt); (h) SF1000N2 (INCI: Dimethicone (2 cSt); and DM-Fluid- 5cSt (INCI: Dimethicone).

Most prefered volatile silicones are (a) decamethyl cyclopentasiloxane (commercially known as D₅ available from Dow Corning DC245; (b) DM-Fluid-2cs; and (c) DM-Fluid -5 cst (INCI: Dimethicone). The most preferred volatile silicone is decamethyl cyclopentasiloxane.

The composition preferably comprises 5 to 90 %, more preferably 10 to 80% water by weight of the composition.

Other useful ingredients usually included in sunscreen compositions may be added for additional benefits. Inorganic sunblocks may be preferably used in the present invention. These include, for example, zinc oxide, iron oxide, silica, such as fumed silica, or titanium dioxide. The total amount of sun block that is preferably incorporated in the composition according to the invention is from 0.1 to 10 % by weight of the composition.

The composition of the invention may additionally comprise a skin lightening agent. The skin lightening agent is preferably chosen from a vitamin B3 compound or its derivative e.g. niacin, nicotinic acid, niacinamide or other well known skin lightening agents e.g. aloe extract, ammonium lactate, azelaic acid, kojic acid, citrate esters, ellagic acid, glycolic acid, green tea extract, hydroquinone, lemon extract, linoleic acid, magnesium ascorbyl phosphate, vitamins like vitamin B6, vitamin B12, vitamin C, vitamin A, a dicarboxylic acid, resorcinol derivatives, hydroxycarboxylic acid like lactic acid and their salts e.g. sodium lactate, and mixtures thereof. Vitamin B3 compound or its derivative e.g. niacin, nicotinic acid, niacinamide are the more preferred skin lightening agent as per the invention, most preferred being niacinamide. Niacinamide, when used, is preferably present in an amount in the range of 0.1 to 10 %, more preferably 0.2 to 5 % by weight of the composition.

The composition according to the invention may also comprise other diluents. The diluents act as a dispersant or carrier for other materials present in the composition, so as to facilitate their distribution when the composition is applied to the skin. Diluents other than water can include liquid or solid emollients, solvents, humectants, thickeners and powders.

The composition of the invention may comprise a conventional deodourant base as the cosmetically acceptable carrier. By a deodorant is meant a product in the stick, or roll-on, which are used for personal deodorant benefit e.g. application in the under-arm or any other area which may or may not contain anti-perspirant actives.

Deodorant compositions can generally be in the form of firm solids, soft solids, gels, creams, and liquids and are dispensed using applicators appropriate to the physical characteristics of the composition.

The compositions of the present invention can comprise a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

According to another aspect of the present invention there is provided use of a composition of the first aspect of the invention for obtaining SPF of at least 20, preferably at least 30 and a UVAPF of at least 6 and preferably at least 10. The use if preferably non-therapeutic.

The invention is now further described by way of the following non-limiting examples.

### Examples

### Examples A and 1: A composition of the invention as compared to that outside the invention

The following compositions as shown in Table -1 were prepared:

| Components | Example A | Example 1 |
|---|---|---|
| OMC (wt%) | 3.75 | 3.75 (entrapped in the silicone elastomer) |
| Silicone Elastomer blend type | DC9041 | Prepared in-house |
| Silicone elastomer blend (wt%) | 21.25 | 21.25 |
| Avobenzone | 1.0 | 1.0 |
| Aerosil R805 | - | 1.8 |
| DC8500 | - | 8.0 |
| DC5225C (emulsifier) | 3.0 | 3.0 |
| Caprylic/ Capric triglyceride (emollient) | 6.0 | 6.0 |
| Water | To 100 | To 100 |

| | | |
|---|---|---|
| OMC: Octyl methoxycinnamate sourced from DSM. Avobenzone: 4-t-butyl, 4'-methoxydibenzoylmethane sourced from DSM. R805: A hydrophobically modified fumed silica from Evonik Degussa GmbH. DC8500: is a Bis (C₁₃-C₁₅ alkoxy) PG amodimethicone available from Dow Corning | | |

The process by which the above compositions were prepared are described below:

### Example A:

The aqueous phase components and oil phase components are mixed and prepared separately. The UVA and the UVB sunscreens are incorporated in the oil phase components along with the elastomer blend. The aqueous phase is then gradually added to the oil phase with stirring to form the water-in-oil emulsion. After the emulsion is prepared, it is homogenized with further constant stirring.

### Example 1:

Step 1: The aqueous phase components were prepared in a separate chamber by mixing water with the water soluble ingredients.
Step 2: The UVA sunscreen matrix was prepared by mixing the Avobenzone with with Caprylic / Capric Triglyceride, the amino silicone and the hydrophobically modified fumed silica at the temperature around 50 °C.
Step 3: The UVB sunscreen (OMC) was entrapped in the silicone elastomer using the typical process described earlier. In short, the following ingredients were taken and mixed thoroughly to form a homogeneous fluid blend at room temperature: silicone hydride copolymer, vinyl terminated silicone polymer, the volatile silicone, the UVB sunscreen. The temperature of the mixture was then raised to about 35 °C and then the desired (platinum based) catalyst was added with constant stirring. The reaction mixture was then heated to about 65 °C with constant stirring until the reaction is completed and the primary elastomer entrapped with the UVB sunscreen is prepared, generally in about an hour, after which the mixture was cooled to room temperature (of about 25 °C). Finally the elastomer was diluted with desired amount of volatile silicone at room temperature to a gel composition with desired rheological property.
Step 4: The oil phase components were prepared by blending the oily ingredients with the silicone oils and the elastomer blend prepared in step 3 above.
Step 5. The water-in-oil emulsion was prepared by adding the aqueous phase components made in step 1 to the oil phase components prepared in step 4 above with constant stirring.
Step 6. The UVA sunscreen matrix generated in step 2 was then added to the emulsion of step 5 with further mixing or homogenizing until a uniform emulsion with desired rheology was obtained.

Invitro-SPF of the various samples was measured using a SPF-290S Analyzer, from Optometrics. The substrate used was a 5 x 5 cm² PMMA slide. The sample was applied at 2 mg/cm² and the measurement was taken over 9 spots to calculate the average SPF value. A 16S-300-009 UVA+UVB simulator, from Solar Light, was used to generate MEDs (minimal erythmal dose) and FDA broad spectrum test method was used to measure UVASPF and to calculate MEDs (1 MED is equivalent to 200 J/m²).

The average SPF and UVAPF for the two samples is give below in Table - 2.

**Table 2**

| Measurements | Example A | Example 1 |
|---|---|---|
| SPF | 17 | 37 |
| UVAPF | 5 | 11 |

The data in table 1 indicates that the composition as per the invention (Examples 1) provides for much higher SPF and UVAPF values as compared to a conventional composition (Example A).

## Claims

1. A photo-protective personal care composition comprising
(a) 0.1 to 10 % by weight of the composition of a UVA sunscreen of the dibenzoylmethane class entrapped in a matrix comprising hydrophobically modified silica and amino functionalized silicone, wherein the UVA sunscreen of the dibenzoylmethane class is 4-tert-butyl-4'-methoxydibenzoylmethane.; and
(b) 0.1 to 10 % by weight of the composition of a UVB sunscreen of the cinnamate class entrapped in a gel comprising silicone elastomer and a volatile silicone, wherein the UVB sunscreen of the cinnamate class is 2-ethylhexyl-p-methoxycinnamate.;
wherein the hydrophobically modified silica has the chemical structure 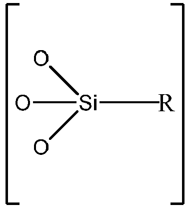
in which R is a C₄ to C₁₈ alkyl group;
wherein the volatile silicone is selected from selected from octa methyl cyclo tetra siloxane, deca methyl cyclo penta siloxane, dodecamethyl cyclo hexa siloxane, or blends of methyl trimethicone and dimethicone and mixtures thereof.

2. A composition as claimed in claim 1 wherein said gel comprising the silicone elastomer and the volatile silicone is prepared by a process of polymerization which comprises the steps of (a) mixing a hydride silicone copolymer with a vinyl terminated silicone polymer in the presence of the volatile silicone compound and a transition metal catalyst and (b) heating the mixture to a temperature of 40 to 90 °C to form the desired gel.

3. A composition as claimed in claim 2 wherein the UVB sunscreen of the cinnamate class is included in step (a) or step (b) before the end of the polymerization process.

4. A composition as claimed in any one of the preceding claims comprising 3 to 40% silicone elastomer by weight of the composition.

5. A composition as claimed in any one of the preceding claims wherein the volatile silicone has a vapor pressure value at 25°C of 2.6 to 1400 Pa, and a heat of vaporization value of 21 to 84 kJ/mole.

6. A composition as claimed in any one of the preceding claims comprising 3 to 60% volatile silicone by weight of the composition.

7. A composition as claimed in any one of the preceding claims comprising 0.5 to 8% hydrophobically modified silica by weight of the composition.

8. A composition as claimed in any one of the preceding claims comprising 1 to 15% amino functionalized silicone by weight of the composition.

9. A method of enhancing the stability of 4-tert-butyl-4'-methoxydibenzoylmethane in a composition comprising 2-ethylhexyl-p-methoxycinnamate comprising the steps of
(i) entrapping 4-tert-butyl-4'-methoxydibenzoylmethane in a matrix comprising hydrophobically modified silica and amino functionalized silicone;
(ii) entrapping 2-ethylhexyl-p-methoxycinnamate in a gel comprising silicone elastomer and a volatile silicone; and
(iii) mixing said matrix with said gel.

10. A composition as claimed in any one of the preceding claims 1 to 8 for use in obtaining an SPF of at least 20.

11. A composition as claimed in any one of the preceding claims 1 to 8 for use in obtaining a UVAPF of at least 6.

## Patentansprüche

1. Körperpflegezusammensetzung mit Lichtschutz, umfassend
(a) 0,1 bis 10 Gewichts-% der Zusammensetzung an einem UVA-Sonnenschutzmittel der Dibenzoylmethan-Klasse, eingeschlossen in einer Matrix, die hydrophob modifiziertes Siliciumdioxid und amino-funktionalisiertes Silikon umfasst, wobei das UVA-Sonnenschutzmittel der Dibenzoylmethan-Klasse 4-tert-Butyl-4'-methoxydibenzoylmethan ist, und
(b) 0,1 bis 10 Gewichts-% der Zusammensetzung an einem UVB-Sonnenschutzmittel der Cinnamat-Klasse, eingeschlossen in einem Gel, das Silikon-Elastomer und ein flüchtiges Silikon umfasst, wobei das UVB-Sonnenschutzmittel der Cinnamat-Klasse 2-Ethylhexyl-p-methoxycinnamat ist,
wobei das hydrophob modifizierte Siliciumdioxid die chemische Struktur 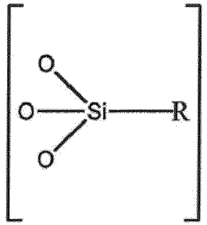
aufweist, in welcher R eine C₄- bis C₁₈-Alkyl-Gruppe ist,
wobei das flüchtige Silikon aus Octamethylcyclotetrasiloxan, Decamethylcyclopentasiloxan, Dodecamethylcyclohexasiloxan oder Mischungen von Methyltrimethicone und Dimethicone und Mischungen davon ausgewählt ist.

2. Zusammensetzung, wie im Anspruch 1 beansprucht, wobei das Gel, das das Silikon-Elastomer und das flüchtige Silikon umfasst, durch ein Polymerisationsverfahren hergestellt wird, das folgende Schritte umfasst: (a) Mischen eines Hydrid-Silikon-Copolymers mit einem Vinyl-terminierten SilikonPolymer in Gegenwart der flüchtigen Silikon-Verbindung und eines Übergangsmetallkatalysators und (b) Erwärmen der Mischung auf eine Temperatur von 40 bis 90°C, um das gewünschte Gel zu bilden.

3. Zusammensetzung, wie im Anspruch 2 beansprucht, wobei das UVB-Sonnenschutzmittel der Cinnamat-Klasse vor dem Ende des Polymerisationsverfahrens in den Schritt (a) oder Schritt (b) einbezogen wird.

4. Zusammensetzung, wie in irgendeinem der vorhergehenden Ansprüche beansprucht, umfassend 3 bis 40% Silikon-Elastomer, bezogen auf das Gewicht der Zusammensetzung.

5. Zusammensetzung, wie in irgendeinem der vorhergehenden Ansprüche beansprucht, wobei das flüchtige Silikon bei 25°C einen Dampfdruckwert von 2,6 bis 1400 Pa und einen Verdampfungswärmewert von 21 bis 84 kJ/Mol aufweist.

6. Zusammensetzung, wie in irgendeinem der vorhergehenden Ansprüche beansprucht, umfassend 3 bis 60% flüchtiges Silikon, bezogen auf das Gewicht der Zusammensetzung.

7. Zusammensetzung, wie in irgendeinem der vorhergehenden Ansprüche beansprucht, umfassend 0,5 bis 8% hydrophob modifiziertes Siliciumdioxid, bezogen auf das Gewicht der Zusammensetzung.

8. Zusammensetzung, wie in irgendeinem der vorhergehenden Ansprüche beansprucht, umfassend 1 bis 15% Amino-funktionalisiertes Silikon, bezogen auf das Gewicht der Zusammensetzung.

9. Verfahren zum Verbessern der Stabilität von 4-tert-Butyl-4'-methoxydibenzoylmethan in einer Zusammensetzung, die 2-Ethylhexyl-p-methoxycinnamat umfasst, umfassend die Schritte:
(i) Einschließen von 4-tert-Butyl-4'-methoxydibenzoylmethan in eine Matrix, umfassend hydrophob modifiziertes Siliciumdioxid und Amino-funktionalisiertes Silikon,
(ii) Einschließen von 2-Ethylhexyl-p-methoxycinnamat in ein Gel, umfassend Silikon-Elastomer und ein flüchtiges Silikon, und
(iii) Mischen der Matrix mit dem Gel.

10. Zusammensetzung, wie in irgendeinem der vorhergehenden Ansprüche 1 bis 8 beansprucht, zur Verwendung zum Erhalten eines SPF von mindestens 20.

11. Zusammensetzung, wie in irgendeinem der vorhergehenden Ansprüche 1 bis 8 beansprucht, zur Verwendung zum Erhalten eines UVA-PF von mindestens 6.

## Revendications

1. Composition pour les soins personnels photoprotectrice comprenant
(a) 0,1 à 10 % en poids de la composition d'un écran solaire contre les UVA de la classe des dibenzoylméthanes piégé dans une matrice comprenant une silice modifiée de manière hydrophobe et un silicone amino-fonctionnalisé, où l'écran solaire contre les UVA de la classe des dibenzoylméthanes est le 4-tert-butyl-4'-méthoxydibenzoylméthane ; et
(b) 0,1 à 10 % en poids de la composition d'un écran solaire contre les UVB de la classe des cinnamates piégé dans un gel comprenant un élastomère de silicone et un silicone volatil, où l'écran solaire contre les UVB de la classe des cinnamates est le p-méthoxycinnamate de 2-éthylhexyle ;
où la silice modifiée de manière hydrophobe a la structure chimique 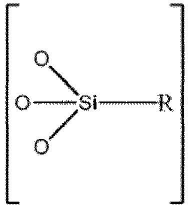
dans laquelle R est un groupe C₄ à C₁₈ alkyle ;
où le silicone volatil est choisi parmi l'octaméthylcyclotétrasiloxane, le décaméthylcyclopentasiloxane, le dodécaméthylcyclohexasiloxane, ou les mélanges de méthyltriméthicone et de diméthicone et leurs mélanges.

2. Composition selon la revendication 1 où ledit gel comprenant l'élastomère de silicone et le silicone volatil est préparé par un processus de polymérisation qui comprend les étapes de (a) mélange d'un copolymère de silicone hydrure avec un polymère de silicone à terminaisons vinyle en présence du composé de silicone volatil et d'un catalyseur à métal de transition et (b) chauffage du mélange à une température de 40 à 90°C pour former le gel souhaité.

3. Composition selon la revendication 2 où l'écran solaire contre les UVB de la classe des cinnamates est inclus dans l'étape (a) ou l'étape (b) avant la fin du processus de polymérisation.

4. Composition selon l'une quelconque des revendications précédentes comprenant 3 à 40 % d'élastomère de silicone en poids de la composition.

5. Composition selon l'une quelconque des revendications précédentes où le silicone volatil a une valeur de pression de vapeur à 25°C de 2,6 à 1400 Pa, et une valeur de chaleur de vaporisation de 21 à 84 kJ/mole.

6. Composition selon l'une quelconque des revendications précédentes comprenant 3 à 60 % de silicone volatil en poids de la composition.

7. Composition selon l'une quelconque des revendications précédentes comprenant 0,5 à 8 % de silice modifiée de manière hydrophobe en poids de la composition.

8. Composition selon l'une quelconque des revendications précédentes comprenant 1 à 15 % de silicone amino-fonctionnalisé en poids de la composition.

9. Procédé d'augmentation de la stabilité du 4-tert-butyl-4'-méthoxydibenzoylméthane dans une composition comprenant du p-méthoxycinnamate de 2-éthylhexyle comprenant les étapes de
(i) piégeage de 4-tert-butyl-4'-méthoxydibenzoylméthane dans une matrice comprenant une silice modifiée de manière hydrophobe et un silicone amino-fonctionnalisé;
(ii) piégeage de p-méthoxycinnamate de 2-éthylhexyle dans un gel comprenant un élastomère de silicone et un silicone volatil; et
(iii) mélange de ladite matrice avec ledit gel.

10. Composition selon l'une quelconque des revendications 1 à 8 précédentes destinée à être utilisée dans l'obtention d'un FPS d'au moins 20.

11. Composition selon l'une quelconque des revendications 1 à 8 précédentes destinée à être utilisée dans l'obtention d'un FPUVA d'au moins 6.
